# EUROPEAN PATENT APPLICATION

(11) **EP 1 110 502 A2**
(43) Date of publication of application: **27.06.2001**
(21) Application number: 00311370.1
(22) Date of filing: 19.12.2000
(51) Int. Cl.: A61B 5/0402, A61B 5/044, A61B 5/00

(54) **Clinical research workstation**

(30) Priority: 22.12.1999 US 469823
(71) Applicant: GE Marquette Medical Systems, Inc., Milwaukee, Wisconsin 53223 (US)
(72) Inventor: Xue, Quizhen, Germantown, Wisconsin 53022 (US); Reddy, Shankara Bonthu, Cedarburg, Wisconsin 53012 (US)
(74) Representative: Pedder, James Cuthbert

(57) **Abstract**

An apparatus and a method are provided for taking multiple physiological signals (e.g., ECG waveforms) from different sources as input, applying multiple algorithms in its core and generating results which are exported for use in clinical studies and research. The apparatus has a built-in database and a built-in spreadsheet to provide a unified platform for all clinical research in the medical field, including, but not limited to, clinical core laboratory work and high-end clinical research.

## Description

This invention relates to analysis of electrocardiograms and other physiological data.

Currently there is a lack of tools for physicians and biomedical scientists to do research work with a large amount of physiological data acquired by medical devices. For example, tools do not exist for exporting measurements and waveform data from the original files and evaluating the value of new clinical parameters and algorithms such as QT dispersion, T wave alternans, signal averaging, and heart-rate variability. The currently employed manual methods of extracting measurements and data are laborious and time-consuming and they suffer from high intra- and inter-observer variability and poor reproducibility. A semi-automatic system with options for physician's review and editing would greatly facilitate clinical research work in cardiology and other branches of medicine.

The present invention is directed to an apparatus and a method for taking multiple physiological signals from different sources as input, applying multiple algorithms in its core and generating results which are exported for use in clinical studies and research. The apparatus has a built-in database and a built-in spreadsheet to provide a unified platform for all clinical research in the medical field, including, but not limited to, clinical core laboratory work and high-end clinical research.

The preferred embodiment of the invention is a clinical research workstation capable of handling a wide range of physiological signals including, but not limited to, resting electrocardiogram (ECG), ambulatory ECG, stress ECG, signal-averaged ECG, intra-cardiac electrical and hemodynamic signals, pulse oximetry signals, blood pressure signals, cardiac output signals, electroen-cephalogram, electro-oculogram, etc. Analysis of each of these physiological signals is supported in one or more separate modules. The research workstation in accordance with the preferred embodiment is also capable of accepting physiological data from a variety of data sources, such as medical devices and systems, including, but not limited to, electrocardiographs, continuous 12-lead ST segment monitors, Holter recorders, stress ECG systems, defibrillators, patient monitors, home healthcare devices, medical data storage/management systems, etc.

In accordance with the preferred embodiment, the research workstation has the capability to export any user-selected data in many output formats and different configurations. Output data will include, but is not limited to, patient demographic information, measurements and waveform signals of both processed and raw data stored in the data file. In addition to the data stored in the file, many measurements and waveforms will also be generated by processing the stored data in the research workstation. Users can select any combination of measurements from a built-in spreadsheet by highlighting the ones they need. A batch processing can be used to export the patient demographics, measurements and/or waveform data from the whole directory or a selected database from a built-in Open Database Connectivity (ODBC) database.

With various physiological data as input from multiple data sources, the system will be able to evaluate new parameters using different algorithms. For example, some high-risk cardiac disease indicators such as signal-averaged ECG parameters, QT dispersion, T wave alternans, and heart rate variability, all from the same patient, can be evaluated at the same time. Algorithms which can be optionally built into the research workstation include, but are not limited to, the following: (1) new measurements with and without user-defined re-analysis from physiological data including, but not limited to, resting ECG, ambulatory ECG, stress ECG, intra-cardiac electrical and hemodynamic signals, and ECG, pulse oximetry and blood pressure signals from neo-natal, pediatric and adult patient monitors and defibrillators; (2) interpretation and re-analysis of resting ECG; (3) QT dispersion and T wave alternans; (4) multilead vector ECG analysis; (5) signal-averaged ECG processing; (6) ECG mapping and modeling; (7) signal filtering and spectral analysis; and (8) heart-rate variability.

In accordance with a further aspect of the preferred embodiment, the research workstation has a built-in ODBC database (Microsoft Access database). The key parameters are stored automatically into the database, and they can be retrieved, sorted and filtered within the system. With this database, reviewing and editing the measurements and interpretation are very convenient. For example, a few simple operations such as, clicking "go forward" and "go backward" buttons will lead the researcher through physiological data files one by one.

In addition, the system has a built-in spreadsheet for selecting for export, reviewing and plotting any of the measurements. The spreadsheet is compatible with standard data analysis software, including, but not limited to, Microsoft Excel and SAS (statistical analysis software) packages, and can be directly saved as a file compatible with standard data analysis software. Researchers can perform most analysis and plotting inside the system, and the spreadsheet interacts with the built-in database seamlessly. A trend of a selected group of physiological parameters/measurements can also be plotted.

The clinical research workstation provides standardized coding/scoring of physiological data, including, but not limited to, Minnesota code, and NOVACODE for resting ECG. The research workstation also provides essential functions needed in core laboratories for clinical studies, including, but not limited to, measuring, reviewing and editing of modifiable time markers in physiological waveforms such as waveform onsets, peaks and offsets, re-analysis based on user-modified markers and serial comparison.

The research workstation software disclosed herein can help physicians advance studies in areas such as disease epidemiology, pharmaceutical research and out-come-based analysis. Using this software, physicians can transform a standard computer with a database program such as Microsoft Access and a spreadsheet program such as Microsoft Excel into an ECG research workstation that allows them to quickly and easily study large volumes of ECG data. The research workstation software enables physicians to store, access, review and plot ECG data with point-and-click efficiency.

The invention will now be described in greater detail, by way of example, with reference to the drawings, in which:-
FIG. 1 is a block diagram showing a conventional personal computer system which can be programmed with research workstation software.
FIG. 2 is a schematic depicting an ECG research workstation window in accordance with the preferred embodiment of the invention.
FIG. 3 is a schematic depicting a Modify window in accordance with the preferred embodiment of the invention.
FIG. 4 and 5 are schematics depicting a 12SL Statement and an array of selectable parameters for export respectively displayed in spreadsheet format in the Results window in accordance with the preferred embodiment of the invention.
FIG. 6 is a schematic depicting a built-in spreadsheet and chart displayed in the Results window.
FIG. 7 is a schematic depicting a Database window in accordance with the preferred embodiment of the invention.
FIG. 8 is a schematic depicting a "Rest ECG Database Select" window in accordance with the preferred embodiment of the invention.
FIG. 9 is a schematic depicting an "Output ECG files in batch mode" window in accordance with the preferred embodiment of the invention.

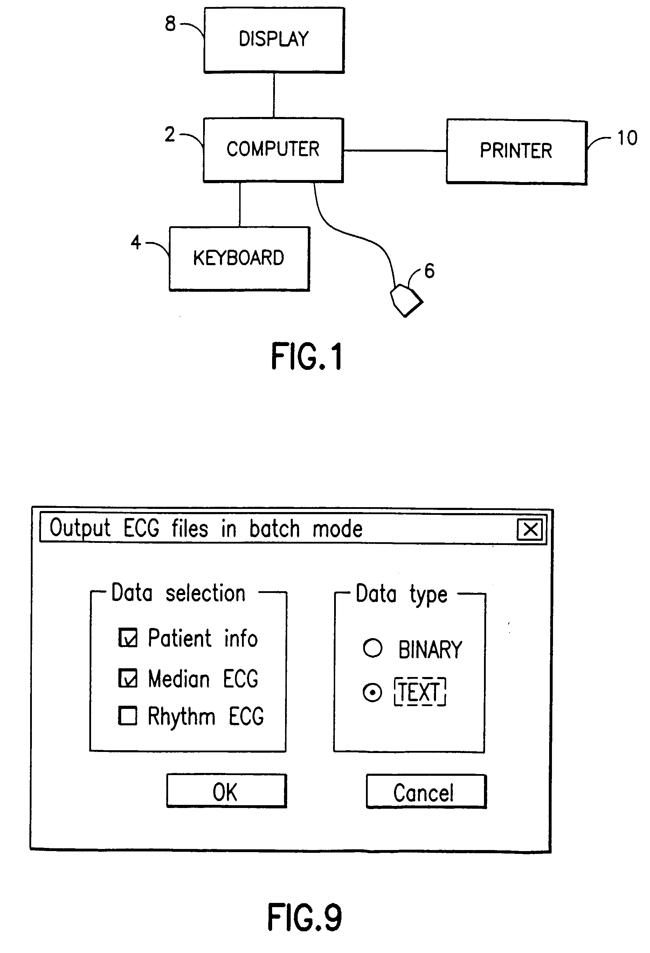

The preferred embodiment of the invention comprises research workstation software installed on a computer system. A typical computer system is generally depicted in FIG. 1 and comprises a computer 2, a keyboard 4, a mouse 6, a display monitor 8 and a printer 10. The software of the preferred embodiment requires the following software: an operating system such as Microsoft Windows 95 or 98, or Windows NT 3.51 or 4.0; a database program such as Microsoft Access for Windows; and a spreadsheet program such as Microsoft Excel for Windows. Although the work-station of the invention can be used to perform clinical research on physiological data including, but not limited to, resting ECG, ambulatory ECG, stress ECG, intra-cardiac electrical and hemodynamic signals, and ECG, pulse oximetry and blood pressure signals, the preferred embodiment will be disclosed in the context of ECG signals for the sake of simplicity, with the understanding that other types of physiological data can be processed in a similar manner. The ECG research workstation incorporates ECG analysis programs such as 12SL from GE Marquette Medical Systems, Inc., which is a computer program for analyzing simultaneously acquired 12-lead ECGs. It makes precise measurements of recorded cardiac signals, then provides an interpretation of the ECG waveforms using ECG interpretation criteria for both rhythm and morphology.

The clinical research workstation software in accordance with the preferred embodiment is used to review and export ECG waveforms, interpretations and measurements for research purposes. The main functions of the research workstation software include: (1) acquiring ECG files from devices and systems; (2) reviewing the ECG waveforms, interpretation and measurements; (3) selecting the ECGs based on user-defined criteria (e.g., age, gender, measurements, interpretation); (4) re-measuring and re-analyzing stored ECGs; and (5) exporting the analysis, measurements and waveform data from the stored ECG files or re-measured/re-analyzed results in a user-selected format.

As used herein, the term "database" includes patient information, parameter data and path names for locating raw ECG (actual waveform) files stored in memory. In addition, the terms "record" and "file" will be used interchangeably.

The terms "class" and "group" will be used interchangeably to refer to groups of records/files in the database.

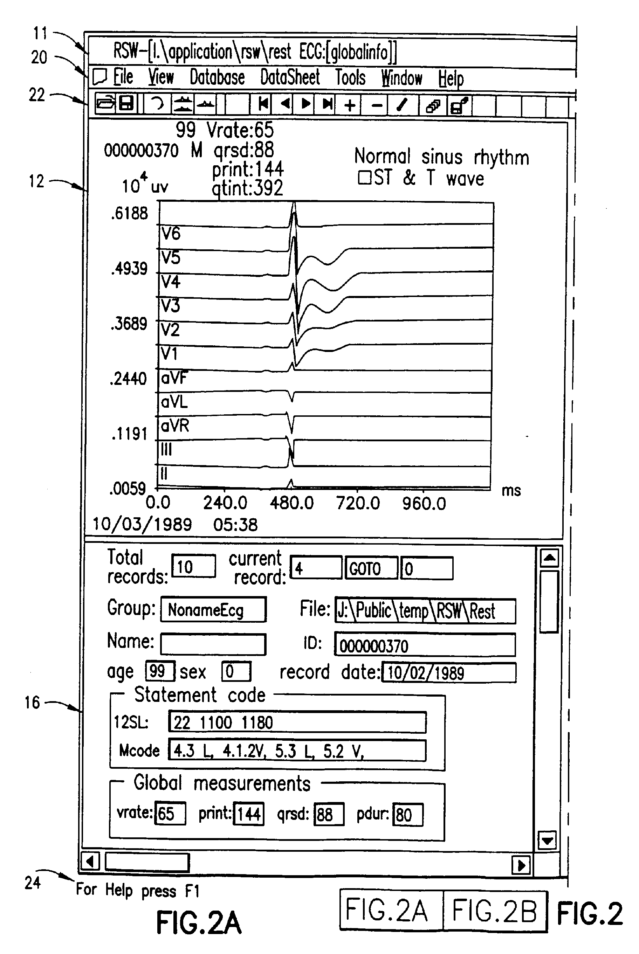

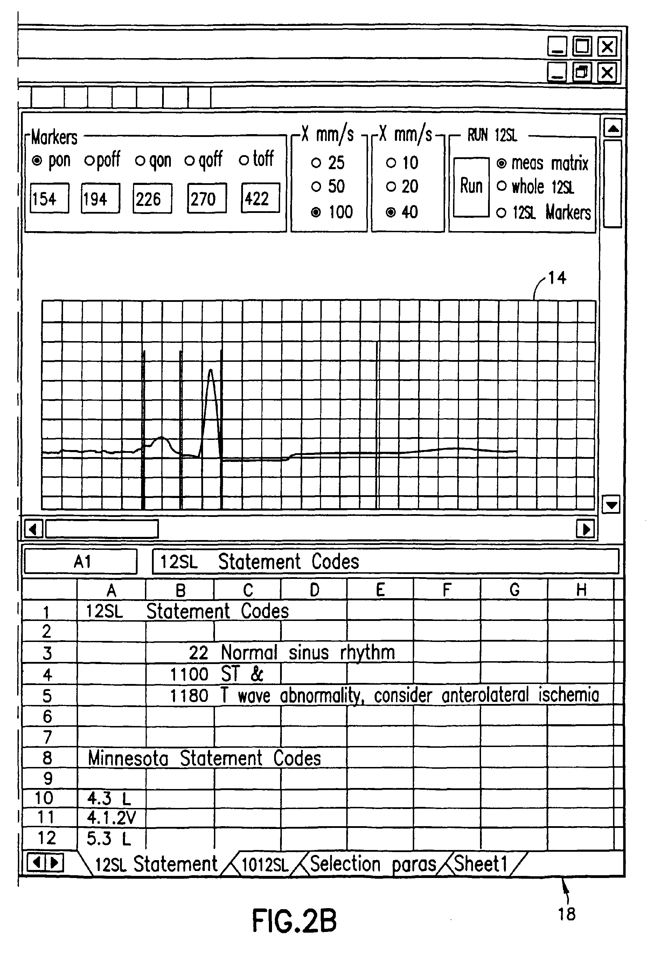

An ECG research workstation window is presented in FIG. 2, showing the window as it appears on the computer display screen when the computer is running the research workstation software of the preferred embodiment. The title bar 11 contains the title name for the ECG research workstation work area, and the close, maximize and minimize buttons for the ECG research workstation window. As seen in FIG. 2, the window is divided into four quadrants: the upper left-hand quadrant is the Signal window 12, the upper right-hand quadrant is the Modify window 14, the lower left-hand quadrant is the Database window 16, and the lower right-hand quadrant is the Results window 18. The windows can be resized by placing the cursor over the intersection of the window borders or over a horizontal or vertical window border and then dragging the cursor using the mouse 6. The Signal window 12 displays 12-lead ECG data as a standard, median or rhythm strip. The Modify window 14 displays and modifies the appearance of a selected signal from the Signal window 12. The Database window 16 displays the records of the Microsoft Access database. With the Database window active, the user can: (1) perform 12SL re-analysis on a group of ECGs automatically; (2) save the results to the database; and (3) review the data. The Results window 18 displays 12SL results and parameters. The menu bar 20 displays pulldown menu options. The available pulldown menus include File, View, Database, DataSheet, Windows and Help menus. The tool bar 22 provides quick access to commonly used features. The buttons (i.e., tool icons) on the tool bar are available depending upon the function being performed and which window is active. Most of these functions are also available from the pulldown menus. The status bar 24 displays research workstation status information.

The tool bar 22 preferably includes an Open button which opens an ECG file and a Save button which saves the current ECG file. ECG files can also be opened and saved by clicking on Open and Save selections in the File menu.

When an ECG file is opened, the ECG waveforms appear in the signal window 12, a selected one of those displayed wave-forms appears in the Modify window 14, the raw waveform data is processed using the ECG analysis program in accordance with the option selected in the Modify window, and the analysis results are displayed in spreadsheet format in the Results window 18. Major parameters such as patient name, ID, and age appear in the Database window 16 when an ECG file is opened.

As seen in FIG. 2, the Signal window 12 appears in the upper left-hand corner of the ECG research workstation window. It displays the raw, unprocessed waveforms. When the Signal window 12 is active, the ECG signals from an opened ECG file can be displayed in one of three formats, the format being selectable by clicking on a corresponding button on the tool bar menu 22, i.e., the Standard ECG Plot, Median ECGs and Rhythm ECGs buttons. The Standard ECG Plot button displays a standard ECG in the Signal window, which is in the format of 2.5 sec by 4, plus 10 sec of lead II and v.1. The Median ECGs button displays the median beats formed by the ECG analysis program. The Rhythm ECGs button displays the 10-sec 12-lead rhythm ECG in the Signal window.

An individual waveform displayed in the Signal window 12 can be selected for display in the Modify window 14. An individual waveform is selected by clicking on it, or by using the up and down keys on the keyboard 4. Pressing the **PgUp** key selects the first waveform; pressing the **PgDn** key selects the last waveform. A selection bar (not shown), appearing in the form of a vertical line, is used to select a specific point in time on the ECG waveform displayed in the Signal window 12. The selection bar can also be used to take measurements. Whenever the selection bar in the Signal window 12 is moved, data changes in the status bar 24 to reflect the new position. The computerized analysis, measurement and interpretation information is provided by the ECG analysis program.

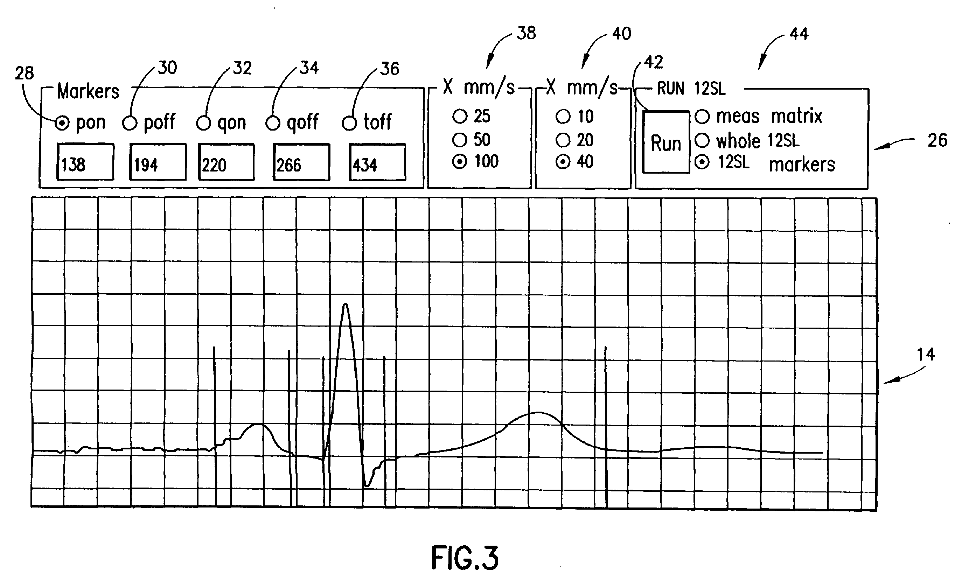

The Modify window 14 provides individual lead waveform demarcation information and variable display gains, and allows for manual manipulation of automatic demarcation points and complete or partial reanalysis of the ECG data by the ECG analysis program. The specific lead displayed in this window is selected from the Signal window, as previously described. A Modify window tool bar 26 is displayed directly above the displayed signal, as seen in FIG. 3. The Modify window includes a multiplicity of virtual radio buttons. The radio button 28 (labeled "pon") is used to modify the P onset markers; the radio button 30 (labeled "poff") is used to modify the P offset markers; the radio button 32 (labeled "qon") is used to modify the Q onset markers; the radio button 34 (labeled "qoff") is used to modify the Q offset markers; and the radio button 36 (labeled "toff") is used to modify the T offset marker. These markers appear as green lines on the ECG signal in the Modify window. The procedure for modifying these markers is as follows: (1) in the Modify window tool bar, click on the virtual radio button corresponding to the marker to be modified; and (2) drag the corresponding green line to the desired location.

In addition, the Modify window tool bar can be used to modify the time and/or amplitude resolution for the signal being displayed in the Modify window. One set 38 of virtual radio buttons is provided for changing the time resolution along the X axis; another set 40 of virtual radio buttons is provided for changing the amplitude resolution along the Y axis. The three radio buttons in set 38 correspond respectively to time resolutions of 25, 50 and 100 mm/sec. The three radio buttons in set 40 correspond respectively to amplitude resolutions of 10, 20 and 40 mm/mV. The resolution can be changed by clicking the desired resolution for either the X or Y axis or for both. In response, the signal changes to conform to the new resolutions. The grids in the Modify window do not change size.

Clicking on the Run button 42 runs the ECG analysis program in accordance with a selected one of three options: (1) for the option labeled "meas matrix", only part of the ECG analysis program is performed; (2) for the option labeled "whole 12SL", the whole ECG analysis is performed, generating more than 700 different ECG measurements; and (3) for the option labeled "12SL markers", the ECG analysis program reanalyzes using the modified markers. When an ECG file is opened, the raw ECG data will be automatically processed in accordance with the option previously selected in the Modify window, i.e., without the need to click on the Run button 42. The results of the analysis are automatically displayed as a spreadsheet in the Results window 18. The desired option is selected by clicking on the corresponding virtual radio button in the set 44 in the Modify window tool bar. For example, to run the ECG analysis program with modified markers, the following steps are performed: (1) click in the Modify window to activate it; (2) for each marker to be modified, click on the corresponding radio button in the Modify window tool bar and then drag the corresponding green line to the desired location; (3) select the "12SL Markers" radio button in set 44 in the Modify window tool bar; and (4) click the Run button 42. The results are displayed in the Results window 18, where they can be reviewed by the researcher.

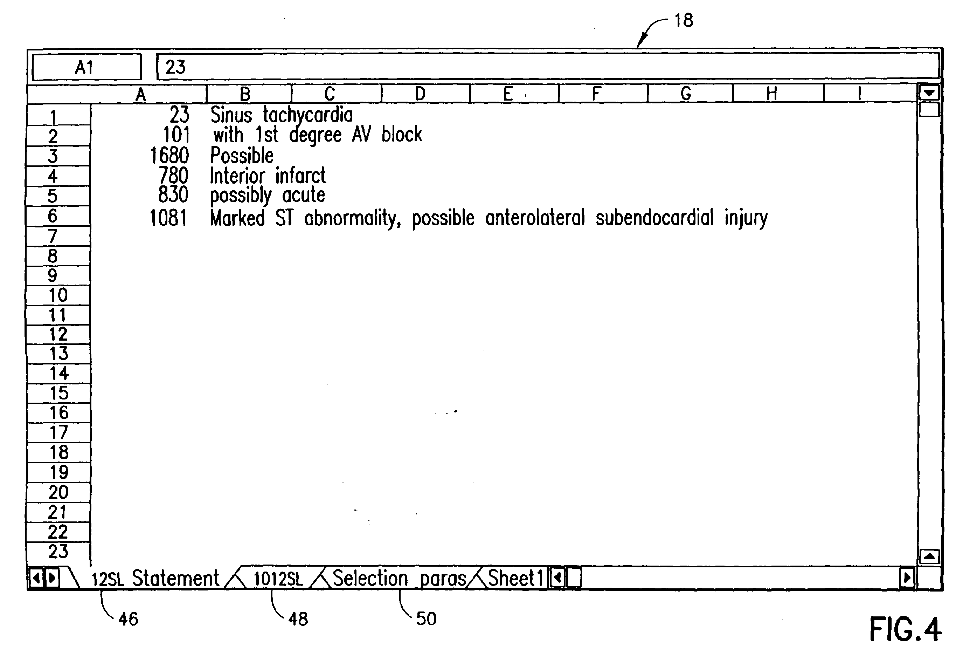

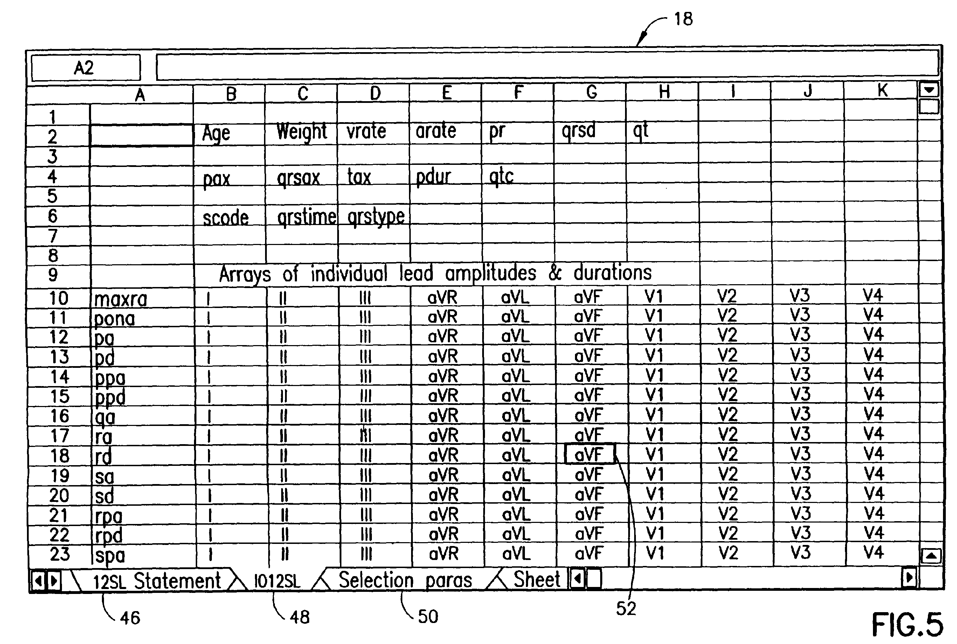

The Results window 18 appears in the lower right-hand comer of the ECG research workstation window. To view the ECG interpretation statement(s) for the selected patient, the researcher clicks the "12SL Statement" tab 46 in the Results window. The statement and its corresponding code are displayed as shown in FIG. 4. When the "IO12SL" tab 48 is activated, the Results window displays the measurements from resting ECGs and patient file parameters for exporting. The data display format can be selected either via buttons on the tool bar 22 or via items listed on a Data Sheet menu (not shown in the drawings). Selection of Sheet on the DataSheet menu displays the exported parameters file as a spreadsheet in the Results window 18. Selection of Chart on the DataSheet menu displays the parameter data in the form of a chart. The Results window can also be used to select parameters for export. In the export parameters mode, the Results window 18 displays an array of selectable parameters, including an array of individual lead amplitudes and durations corresponding to the waveforms displayed in the Signal window 12. To view the window shown in FIG. 5, the user clicks on Select Parameters in the Data Sheet menu or a corresponding button in the tool bar 22. This allows the user to select parameters to store in an external file by clicking on the desired parameters. The selected parameters are highlight-ed. In the example shown in FIG. 5, the selected parameter is indicated by the surrounding rectangular box 52. In response to clicking on Done Parameters Selection! in the Data Sheet menu, a dialog box (not shown) appears on the display, asking the user whether the parameters should be exported now. The user clicks Yes to save the file. Then a Save As dialog box (not shown) appears. The user then selects the appropriate directory location and types in the file name. The user then clicks Save to save the file. A Select ECG Source box (not shown) appears having two virtual radio buttons, one labeled "From whole directory" and the other labeled "From ECG database". Selecting "From whole directory" will extract the ECG parameters from all folders in a selected directory. Selecting "From ECG database" will extract the ECG parameters from a selected database class. The ECG parameters are saved when an OK button in the Select ECG Source is clicked on. The exported parameters file can be viewed through the Selection Paras tab 50 in the Results window, e.g., in response to selection of Read Parameters in the DataSheet menu.

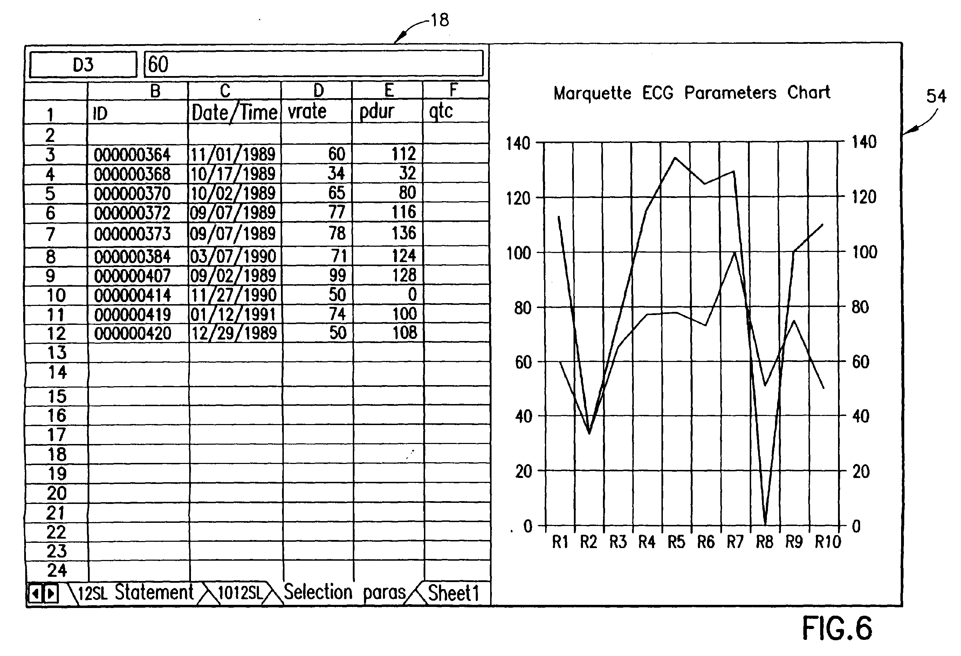

In addition, parameters can be viewed in chart form by linking the spreadsheet data to a chart. The procedure for viewing parameters in chart form is as follows: (1) highlight the region in the Results window which is to be linked to a chart; (2) select Link Chart to Sheet on the Data Sheet menu to toggle the linkage between the chart values and the chart on; and (3) select Chart on the Data Sheet menu to toggle the chart display on. The selected data appears in chart form in the Results window 18, as shown by representative chart 54 in FIG. 6.

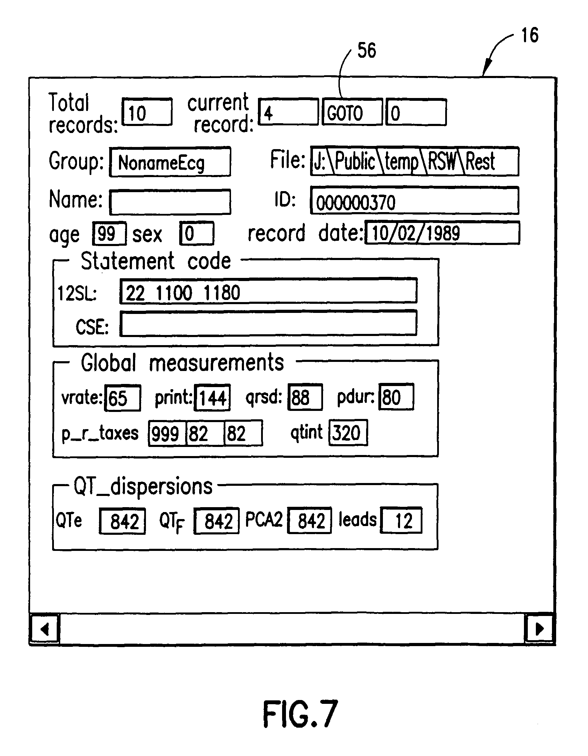

The Database window 16 appears in the lower left-hand corner of the ECG research workstation window. As shown in FIG. 7, the Database window displays information contained in the database. As previously noted, the database includes the path names to raw ECG data files, not the actual waveforms themselves. In accordance with the preferred embodiment of the invention, the Database window comprises the following fields:
- Group: Used to categorize ECGs in the database.
- File: Path to the directory storing the raw ECG file.
- Name: Patient name.
- ID: Patient identification number.
- Age: Patient age.
- Sex: Patient gender.
- Record time: Time the ECG was taken.
- Under the frame labeled "Statement code":
   ◆ 12SL: ECG interpretation statement codes.
   ◆ CSE: Special coding, Minnesota coding.
- Under the frame labeled "Global measurements":
   ◆ Vrate: Ventricular rate.
   ◆ PRint: PR interval.
   ◆ qrsd: QRS duration.
   ◆ Pdur: P duration.
   ◆ P-R-T axes: P, R and T axes.
   ◆ QTint: QT interval.
- Under the frame labeled "QT dispersions":
   ◆ QT-end: from the beginning of the Q wave to the end of the T wave.
   ◆ QT-peak: from the beginning of the Q wave to the peak of the T wave.
   ◆ PCAS2: Use principal component analysis to describe T wave morphology.
   ◆ Leads: Number of leads
- Total records: The number of records used in calculating QT dispersion.
- Current record: The order number of the current record in the database.

To display a specific record in the Signal window, the user must enter the number of the record in the field next to the GOTO button 56 (see FIG. 4). Initially, the record having number 1 is displayed. Thereafter the system user can scroll through the records of the particular database group identified in the Group field by clicking on a Next Record button in the tool bar 22 (see FIG. 1). The toolbar also includes buttons for adding, deleting and modifying records in the database. During scrolling, the data in all of the windows changes to correspond to the new record, i.e., the waveforms for the next record are displayed in the Signal window, a selected one of those displayed waveforms is displayed in the Modify window, the computed parameters for those displayed waveforms are displayed in the Results window, and the patient information, Statement code, Global measurements and QT dispersions data fields in the Database window are updated. As used herein, the term "global" means that they are measured across all leads. The QT dispersion can be automatically calculated by the computer using any well-known algorithm, such as the algorithms disclosed in U.S. Patent No. 5,792,065 and in the article by Xue et al. entitled "Algorithms for Computerized QT Analysis," J. Electrocardiology, Vol. 30 Supplement.

The database tool bar is located in the left side of the tool bar 22. The buttons of the database tool bar are available only when the Database window is active. The database tool bar includes buttons for: displaying a record of the database; adding a record to the end of the database; deleting a record from the database; replacing a current record with a modified version. In addition, the database tool bar has buttons for saving files in a whole directory to a database; performing a database search; batch exporting of patient information and/or ECG data; and batch exporting selected parameters to an external file from a selected database. When the database tool bar is active, its buttons are green. In addition, a Database menu is provided having selections corresponding to the four functions (respectively named Read Files to Database, Select Database, Export Data and Export Parameters) set forth in the next preceding sentence of this paragraph.

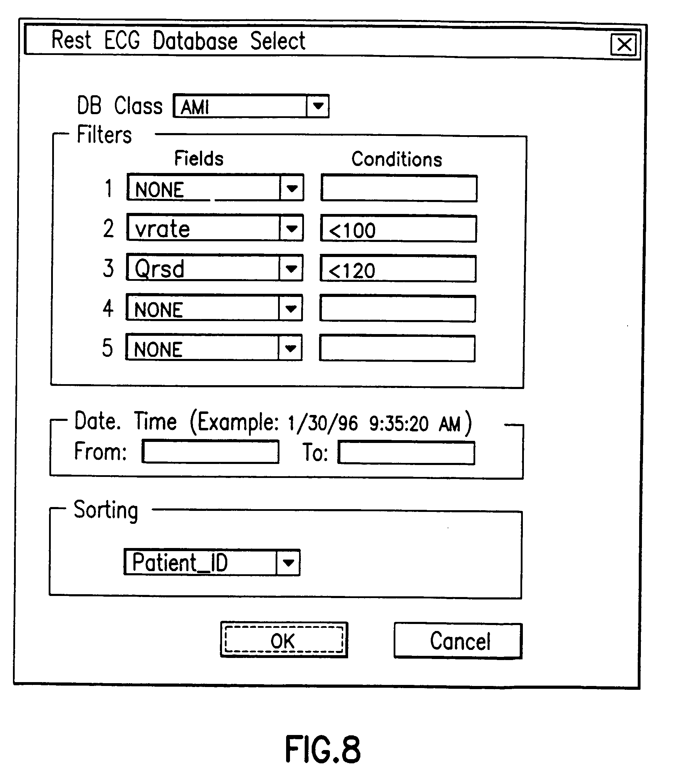

When the Database window is active, any group of database records can be loaded in working memory and reviewed by the user. Alternatively, the user may construct a new database group by defining a set of search parameters and then filtering out all data lying outside those parameters. Querying the database is useful in finding ECGs that match certain criteria. A search for data satisfying these conditions can be performed as follows: (1) Click inside the Database window to activate it. (2) Click Select Database from the Database menu. The Rest ECG Database Select window (shown in FIG. 8) appears. This window is used when searching the database for records matching specified criteria. (3) If desired, select a database class from the popup menu. If this field is left blank, then all database records are available for the search. (4) Define the parameters for the ECGs that are sought. There are five filters in the Filters section of the Rest ECG Database Select window. Filter 1 is used if the searcher wants to find a record for a specific patient by name or patient identification number. The remaining filters can be used to set search parameters, e.g., the searcher can search for all ECGs in which the ventricular heart rate is less than 100 and the QRS duration is less than 120 msec. (5) Then the date and time are entered. (6) If sorting of the data is desired, a sorting methodology can be selected from the Sorting popup menu. For example, the data gathered by the search can be sorted according to Patient ID. (7) Click the OK button. The selected records from the database are loaded in working memory and appear in the Database window. The raw ECG waveforms from the first record in the group are displayed in the Signal window; a selected one of those waveforms is displayed in the Modify window; and the parameters generated by the ECG analysis program are displayed in the Results window.

The preferred embodiment also provides for the batch exportation of data using the following procedure: (1) Click inside the Database window to activate it. (2) Click Export Data from the Database menu. The "Output ECG files in batch mode" window (shown in FIG. 9) appears. (3) The user then selects which data he/she wants to export. The selection of "Patient info" will result in the inclusion of patient information, measurements and parameters for the raw ECG files being output. The raw ECG data to be included in the batch can be either median or rhythm ECG signal, depending on which of the corresponding radio buttons is selected. (4) The user then selects whether the data type will be binary data or text. (5) When the user clicks on the OK button in the "Output ECG files in batch mode" window, the Select ECG Source window (previously described) appears. (6) The user selects where the data is to be retrieved from. Selecting "From whole directory" will extract the data from all folders in a selected directory. Selecting "From ECG database" will extract the data from a selected database class. The ECG parameters are saved when an OK button in the Select ECG Source is clicked on.

ECG files may be imported into the workstation by any conventional means, including but not limited to copying ECG files from a diskette, transferring ECG files from a PCMCIA card via local and wide area networks, wireless communication channels, and internet.

For the sake of good order, various features of the invention are set out in the following clauses:-
1. A system comprising a computer (2), a display screen (8) connected to said computer, and an operator interface (4, 6) connected to said computer, said computer comprising memory for storing a multiplicity of raw ECG files and a database comprising a respective record for each of said raw ECG files, each raw ECG file comprising data representing a plurality of ECG waveforms, and each record comprising a patient identifier, a pathname to the corresponding raw ECG file, and data representing measurements taken from said waveforms of said corresponding raw ECG file, wherein said computer is programmed to perform the following steps:
   loading a multiplicity of database records in working memory in response to activation of a load database records function via said operator interface; and
   controlling said display screen to concurrently display first and second windows, said first window (12) displaying a plurality of ECG waveforms from a raw ECG file identified by a pathname in one of said loaded database records, and said second window (16) displaying data from said one database record.
2. The system as recited in clause 1, wherein said second window comprises a first field which is automatically loaded with a patient identifier from said database record, and a second field which is automatically loaded with an ECG interpretation code from said database record.
3. The system as recited in clause 1, wherein said second window comprises a first field which is automatically loaded with a patient identifier from said database record, and a second field which is automatically loaded with a global measurement datum from said database record.
4. The system as recited in clause 1, wherein said second window comprises a first field which is automatically loaded with a patient identifier from said database record, and a second field which is automatically loaded with a QT dispersion datum from said database record.
5. The system as recited in clause 1, wherein said computer is programmed to scroll through said raw ECG files and said database records in tandem in response to activation of a scrolling function via said operator interface, a plurality of ECG waveforms for each successive raw ECG file being successively displayed in said first window and data of a corresponding database record associated with said successive raw ECG file being successively displayed in said second window.
6. The system as recited in clause 5, wherein said computer is programmed to control said display screen to display a toolbar comprising a scrolling icon when said second window is in an active state, said scrolling function being activated by clicking on said scrolling icon using said operator interface.
7. The system as recited in clause 1, wherein said computer is programmed to perform a database search in response to activation of a database search function via said operator interface.
8. The system as recited in clause 7, wherein said computer is further programmed to sort the database search results in accordance with a sorting function selected via said operator interface prior to activation of said database search.
9. The system as recited in clause 7, wherein said computer is further programmed to display a database search menu in response to an operator interface input, said database search menu comprising a field for entry of a database class, and to search only records belonging to said database class if said database class is entered in said field.
10. The system as recited in clause 9, wherein said database search menu further comprises a field for entering a sorting function, said computer being further programmed to sort the database search results in accordance with said entered sorting function.
11. The system as recited in clause 7, wherein said database search comprises the step of constructing a new database group by filtering out all data in said database lying outside a set of search parameters input via said operator interface prior to activation of said database search.
12. The system as recited in clause 11, wherein said filter comprises a patient identifier.
13. The system as recited in clause 11, wherein said filter comprises a range for a global measurement.
14. The system as recited in clause 11, wherein said computer is further programmed to sort the database search results in accordance with a sorting function selected via said operator interface prior to activation of said database search.
15. The system as recited in clause 11, wherein said computer is further programmed to display a database search menu in response to an operator interface input, said database search menu comprising a first field for entry of a parameter name and a second field for entry of a range of values for the parameter identified in said first field, said computer being further programmed to find database records for which said identified parameter has a value falling within said specified range.
16. The system as recited in clause 1, wherein said computer is further programmed to control said display screen to display a third window (18) concurrently with said first and second windows, said third window displaying an array of parameter values in spreadsheet format, said array of parameter values being retrieved from the database record associated with the plurality of ECG waveforms being displayed in said first window.
17. The system as recited in clause 2, wherein said computer is programmed to control said display screen to display a third window (18) concurrently with said first and second windows, said third window displaying a list of ECG analysis statements in conjunction with corresponding ECG interpretation codes, said ECG interpretation codes also being displayed in said second field in said second window.
18. The system as recited in clause 15, wherein said computer is programmed to export a batch of selected parameter values from said database in dependence on an export set of parameter identifiers selected via said operator interface and in response to an export parameters from database instruction input via said operator interface.
19. A research workstation comprising:
   a display screen (8);
   an operator interface (4, 6);
   memory (2) for storing a multiplicity of raw ECG files and a database comprising a respective record for each of said raw ECG files, each raw ECG file comprising data representing a plurality of ECG waveforms, and each record comprising a patient identifier, a pathname to the corre-sponding raw ECG file, and data representing measurements taken from said waveforms of said corresponding raw ECG file;
   means (2) for loading a multiplicity of database records in working memory in response to activation of a load database records function via said operator interface; and
   means (2) for concurrently displaying first and second windows on said display screen, said first window (12) displaying a plurality of ECG waveforms from a raw ECG file identified by a pathname in one of said loaded database records, and said second window (16) displaying data from said one database record.
20. The workstation as recited in clause 19, further comprising means (2) for scrolling through said raw ECG files and said database records in tandem in response to activation of a scrolling function via said operator interface, a plurality of ECG waveforms for each successive raw ECG file being successively displayed in said first window and data of a corresponding database record associated with said successive raw ECG file being successively displayed in said second window.
21. The workstation as recited in clause 19, further comprising means (2) for retrieving selected records from said database in dependence on search conditions input via said operator interface and in response to activation of a database search function via said operator interface, wherein said selected records satisfy said search conditions.
22. The workstation as recited in clause 21, further comprising means (2) for sorting said selected search records in accordance with a sorting function selected via said operator interface.
23. The workstation as recited in clause 19, further comprising means (2) for displaying a third window (18) concurrently with said first and second windows on said display screen, said third window displaying an array of parameter values in spreadsheet format, said array of parameter values being retrieved from the database record associated with the plurality of ECG waveforms being displayed in said first window.
24. The workstation as recited in clause 19, further comprising means (2) for exporting a batch of selected parameter values from said database in dependence on an export set of parameter identifiers selected via said operator interface and in response to an export parameters from database instruction input via said operator inter-face.
25. A method for performing research on a workstation, comprising the steps of:
   storing a multiplicity of raw ECG files, each raw ECG file comprising data representing a plurality of ECG waveforms;
   constructing a database comprising a respective record for each of said raw ECG files, each record comprising a patient identifier, a pathname to the corresponding raw ECG file, and data representing measurements taken from said waveforms of said corresponding raw ECG file;
   loading a selected group of database records in working memory; and
   concurrently displaying a plurality of ECG waveforms from a raw ECG file identified by a pathname in one of said loaded database records and data from said one database record.
26. The method as recited in clause 25, further comprising the step of scrolling through said raw ECG files and said database records in tandem, a plurality of ECG waveforms for each successive raw ECG file and data of a corresponding database record associated with said successive raw ECG file being successively displayed.
27. The method as recited in clause 25, further comprising the steps of:
   selecting a search condition; and
   electronically scanning said database in search of database records which satisfy said search condition.
28. The method as recited in clause 27, wherein said search condition comprises a range of values for a selected parameter.
29. The method as recited in clause 25, further comprising the step of electronically sorting said database records which satisfy said search condition in accordance with a sorting function.

## Claims

1. A system comprising a computer (2), a display screen (8) connected to said computer, and an operator interface (4, 6) connected to said computer, said computer comprising memory for storing a multiplicity of raw ECG files and a database comprising a respective record for each of said raw ECG files, each raw ECG file comprising data representing a plurality of ECG waveforms, and each record comprising a patient identifier, a pathname to the corresponding raw ECG file, and data representing measurements taken from said waveforms of said corresponding raw ECG file, wherein said computer is programmed to perform the following steps:
loading a multiplicity of database records in working memory in response to activation of a load database records function via said operator interface; and
controlling said display screen to concurrently display first and second windows, said first window (12) displaying a plurality of ECG waveforms from a raw ECG file identified by a pathname in one of said loaded database records, and said second window (16) displaying data from said one database record.

2. The system as recited in claim 1, wherein said second window comprises a first field which is automatically loaded with a patient identifier from said database record, and a second field which is automatically loaded with an ECG interpretation code from said database record.

3. The system as recited in claim 1, wherein said second window comprises a first field which is automatically loaded with a patient identifier from said database record, and a second field which is automatically loaded with a global measurement datum from said database record.

4. The system as recited in claim 1, wherein said second window comprises a first field which is automatically loaded with a patient identifier from said database record, and a second field which is automatically loaded with a QT dispersion datum from said database record.

5. A research workstation comprising:
a display screen (8);
an operator interface (4, 6);
memory (2) for storing a multiplicity of raw ECG files and a database comprising a respective record for each of said raw ECG files, each raw ECG file comprising data representing a plurality of ECG waveforms, and each record comprising a patient identifier, a pathname to the corresponding raw ECG file, and data representing measurements taken from said waveforms of said corresponding raw ECG file;
means (2) for loading a multiplicity of database records in working memory in response to activation of a load database records function via said operator interface; and
means (2) for concurrently displaying first and second windows on said display screen, said first window (12) displaying a plurality of ECG waveforms from a raw ECG file identified by a pathname in one of said loaded database records, and said second window (16) displaying data from said one database record.

6. The workstation as recited in claim 5, further comprising means (2) for scrolling through said raw ECG files and said database records in tandem in response to activation of a scrolling function via said operator interface, a plurality of ECG waveforms for each successive raw ECG file being successively displayed in said first window and data of a corresponding database record associated with said successive raw ECG file being successively displayed in said second window.

7. The workstation as recited in claim 5 or 6, further comprising means (2) for retrieving selected records from said database in dependence on search conditions input via said operator interface and in response to activation of a database search function via said operator interface, wherein said selected records satisfy said search conditions.

8. The workstation as recited in claim 7, further comprising means (2) for sorting said selected search records in accordance with a sorting function selected via said operator interface.

9. A method for performing research on a workstation, comprising the steps of:
storing a multiplicity of raw ECG files, each raw ECG file comprising data representing a plurality of ECG waveforms;
constructing a database comprising a respective record for each of said raw ECG files, each record comprising a patient identifier, a pathname to the corresponding raw ECG file, and data representing measurements taken from said waveforms of said corresponding raw ECG file;
loading a selected group of database records in working memory; and
concurrently displaying a plurality of ECG waveforms from a raw ECG file identified by a pathname in one of said loaded database records and data from said one database record.

10. The method as recited in claim 9, further comprising the step of scrolling through said raw ECG files and said database records in tandem, a plurality of ECG waveforms for each successive raw ECG file and data of a corresponding database record associated with said
